# EUROPÄISCHE PATENTSCHRIFT

(11) **EP 0 821 229 B1**
(45) Veröffentlichungstag und Bekanntmachung des Hinweises auf die Patenterteilung: **08.09.2004**
(21) Anmeldenummer: 97110742.0
(22) Anmeldetag: 01.07.1997
(51) Int. Cl.: G01N 27/22

(54) **Gassensor und Verwendung eines Gassensors zum selektiven Nachweis von Kohlenwasserstoffen im Abgastrom von Ottomotoren**
Gas sensor and usage of a gas sensor for the selective detection of hydrocarbons in the exhaust gas of a spark ignition engine
Captuer de gaz et utilisation d'un capteur de gaz pour la détection sélective des hydrocarbones dans des gaz d'echappement dans des moteurs otto

(30) Priorität: 26.07.1996 DE 19630209
(43) Veröffentlichungstag der Anmeldung: 28.01.1998
(73) Patentinhaber: DaimlerChrysler AG, 70567 Stuttgart (DE)
(72) Erfinder: Plog, Carsten, Dr. Dipl.-Phys., 88677 Markdorf (DE); Maunz, Werner, 88677 Markdorf (DE)

(56) Entgegenhaltungen:
- EP-A- 0 426 989
- US-A- 5 296 196
- US-A- 5 357 749

## Beschreibung

Die Erfindung betrifft die Verwendung eines Gassensors zum selektiven Nachweis von Kohlenwasserstoffen (im folgenden auch mit KW abgekürzt) in sauerstoffarmen Gasen.

Für den Nachweis der Wirksamkeit von Abgasnachbehandlungssystemen an Bord von Kraftfahrzeugen mit Otto-Verbrennungsmotoren (OBD) ist es bekannt, Kohlenwasserstoffsensoren einzusetzen. Eine besondere Anforderung an den Kohlenwasserstoff-Sensor ist seine Unempfindlichkeit gegenüber CO und H₂, da beide Komponenten im Abgas von Otto-Motoren - verglichen mit KW - im Überschuß vorliegen.

In der **EP 0 426 989 B1** wird ein selektiver chemischer Sensor für Gase beschrieben, der diese Anforderungen erfüllt. Der Sensor besteht in einer Ausführungsform aus einer platinhaltigen, zeolithischen Schicht auf einer Interdigitalkondensatorstruktur (Interdigitalkondensator wird im folgenden auch mit IDK abgekürzt). In **Sensors and Actuators, B 24-25 (1995) 403-406** wird gezeigt, daß eine auf dieser Basis mit Siebdrucktechnik hergestellter PtNaY-IDK Sensor bei 350 °C an Luft 1000 ppm Butan nachweist, während gleichzeitig weder 1000 ppm CO noch 1000 ppm H₂ detektiert werden.

Als eine weitere, schwierige Anforderung in der OBD-Anwendung ist die gegenüber Atmosphärenbedingung stark verminderte Sauerstoffkonzentration im Bereich von λ=1 anzusehen. Gleichzeitig müssen Kohlenwasserstoffe in Konzentrationen deutlich unter 100 ppm nachgewiesen werden.

Die in den beiden obengenannten Literaturstellen zum Stand der Technik beschriebenen Sensoren behandeln die Detektion von Kohlenwasserstoffen ausschließlich in sauerstoffreichen Gasen, z.B. in Umgebungsluft (Sauerstoffanteil ca. 20 %). Bei hoher Sauerstoffkonzentration werden die Kohlenwasserstoffe und das CO und der Wasserstoff im Verlauf einer katalytischen Totaloxidation vollständig in die thermodynamisch jeweils stabilsten Verbindungen umgesetzt. Im Falle der Kohlenwasserstoffe entsteht CO₂ und H₂O, im Falle von CO entsteht nur CO₂ und im Falle von H₂ entsteht nur H₂O. Die katalytische Aktivität der hier betrachteten edelmetalldotierten Zeolithe ist so hoch, daß H₂ bereits bei Raumtemperatur, CO bei Temperaturen unter 100°C vollständig umgesetzt werden. Dagegen wird für die katalytische Umsetzung der Kohlenwasserstoffe eine deutlich höhere Temperatur benötigt. Bei dieser Betriebstemperatur des KW-Sensors ist die Reaktionsdauer der Totalumsetzung von Kohlenwasserstoffen deutlich höher als die von H₂ und CO. Deshalb wird die Mobilität der Kationen im Zeolithsystem von der länger dauernden Kohlenwasserstoff-Umsetzung genügend stark behindert, so daß sie meßtechnisch erfaßt werden kann, während die Zeitdauer der Umsetzung von H₂ und CO zu kurz ist. Dies ist der Grund, warum Kohlenwasserstoffe von dem KW-Sensor nachgewiesen werden, H₂ und CO aber nicht.

Die Dauer der katalytischen Reaktion, die Ionenleitfähigkeit des Zeolithen bei der Betriebstemperatur des Sensors, die sterischen Verhältnisse des Porensystems der Zeolithe (d.h. der Abstand der kationischen Adsorptionsplätze und der katalytisch wirksamen Zentren) müssen genau aufeinander abgestimmt sein. Dies ist für die Totaloxidation von Kohlenwasserstoffen an den obengenannten edelmetalldotierten Zeolithen bei den verwendeten Betriebstemperaturen und beobachteten Frequenzbereichen der Impedanz gerade der Fall. Für jede andere Reaktion liegen die Verhältnisse jeweils anders.

Im Falle sehr niedriger Sauerstoffgehalte kann anstatt der beschriebenen Totaloxidation nur eine Teiloxidation der Kohlenwasserstoffe auftreten. Dann liegen andere Reaktionszeiten und Reaktionsprodukte vor. Aus diesem Grunde erscheinen die obengenannten, bekannten KW-Sensoren für den Nachweis von Kohlenwasserstoffen mit sehr niedrigen Sauerstoffgehalten, wie sie im ottomotorischen Abgas nach λ=1 vorliegen, nicht geeignet.

Der Erfindung liegt als technisches Problem die Bereitstellung eines Sensors zum selektiven Nachweis von Kohlenwasserstoffen in sauerstoffarmen Gasen (O₂-Anteil kleiner als 10 000 ppm) zugrunde.

Dieses Problem wird durch den Gegenstand des Anspruchs 1 gelöst. Vorteilhafte Ausgestaltungen der Erfindung sind Gegenstand weiterer Ansprüche.

Es wurde überraschenderweise gefunden, daß der aus der **EP 0 426 989 B1** an sich bekannte KW-sensitive Sensor (KW=Kohlenwasserstoff) eine hohe Empfindlichkeit auch bei reduziertem Sauerstoffgehalt besitzt.

Der erfindungsgemäße Sensor umfaßt
- ein als Kondensator wirkendes Bauteil;
- eine gasdurchlässige, sensitive Schicht als Dielektrikum, wobei die sensitive Schicht ein Edelmetall-dotierter Zeolith ist, der eine geordnete, kristalline Struktur aus Primärporen aufweist, deren Durchmesser in der Größenordnung des gaskinetischen Durchmessers der zu detektierenden Gasmoleküle ist.

Dem Sensor liegt folgendes Meßprinzip zugrunde:
Die Impedanz des Sensors ist frequenzabhängig und wird durch die ionische Leitfähigkeit des Zeoliths und deren Beeinflussung durch die Wechselwirkung des Meßgaskomponente mit der inneren Zeolithoberfläche bestimmt. Die katalytische Umsetzung der Kohlenwasserstoffe an den Edelmetallzentren in den Poren der Zeolithe läuft bei der Sensorbetriebstemperatur nicht unendlich schnell, sondern mit einer endlichen Geschwindigkeit ab. Während der Zeitdauer der katalytischen Umsetzung wird die Mobilität der Kationen im elektrischen Feld durch die Reaktion behindert, was im betrachteten Frequenzbereich zu einer Widerstandserhöhung führt. Die Betriebstemperatur des Sensors liegt im Bereich zwischen 300°C und 500°C.

Die Detektion erfolgt durch Bestimmung der Impedanz des Sensors bei einer geeigneten, festen Frequenz.

Als Zeolith wird ein ZSM5-Zeolith verwendet, insbesondere mit einem Modul SiO₂/Al₂O₃ von 20 bis 50.

Als Zeolith-Dotierungen werden Pt oder Pd mit einem Anteil von 0,1 bis 5 Gew.-% verwendet.

Als besonders vorteilhaft hat sich ein ZSM5-Zeolith mit einem Pt-Anteil von 3 Gew.-% im Zeolithpulver erwiesen.

In der folgenden Tabelle ist der erfindungsgemäße Zeolith zusammen mit seinem jeweiligen Porendurchmesser angegeben:

Die Erfindung wird anhand von Fig. näher erläutert. Es zeigen:
- Fig. 1: ein Diagramm zum Sauerstoffeinfluß auf die Empfindlichkeit - relative Kapazitätsänderung dC/C (oben) und relative Widerstandsänderung dR/R (unten) - des erfindungsgemäßen Sensors bei einer KW-Beaufschlagung von 1000 ppm Butan, 5% H₂O, Rest N₂ in Abhängigkeit von der Frequenz,
- Fig. 2: relative Widerstandsänderung dR/R eines erfindungsgemäßen Sensors bei einer KW-Beaufschlagung mit 500 ppm einer Propan/Propen-Mischung bei 0,6% O₂, 5% H₂O, Rest N₂ in Abhängigkeit von der Frequenz,
- Fig. 3: relative Widerstandsänderung dR/R eines erfindungsgemäßen Sensors bei einer KW-Beaufschlagung mit 20 ppm einer Ethan/ Ethen/ Ethin/Propen-Mischung bei 0,6% O₂, 5% H₂O, Rest N₂ in Abhängigkeit von der Frequenz,
- Fig. 4: einen erfindungsgemäßen Sensor, ausgebildet als Interdigitalkondensatorstruktur mit darauf angebrachter Zeolithschicht als Dielektrikum.

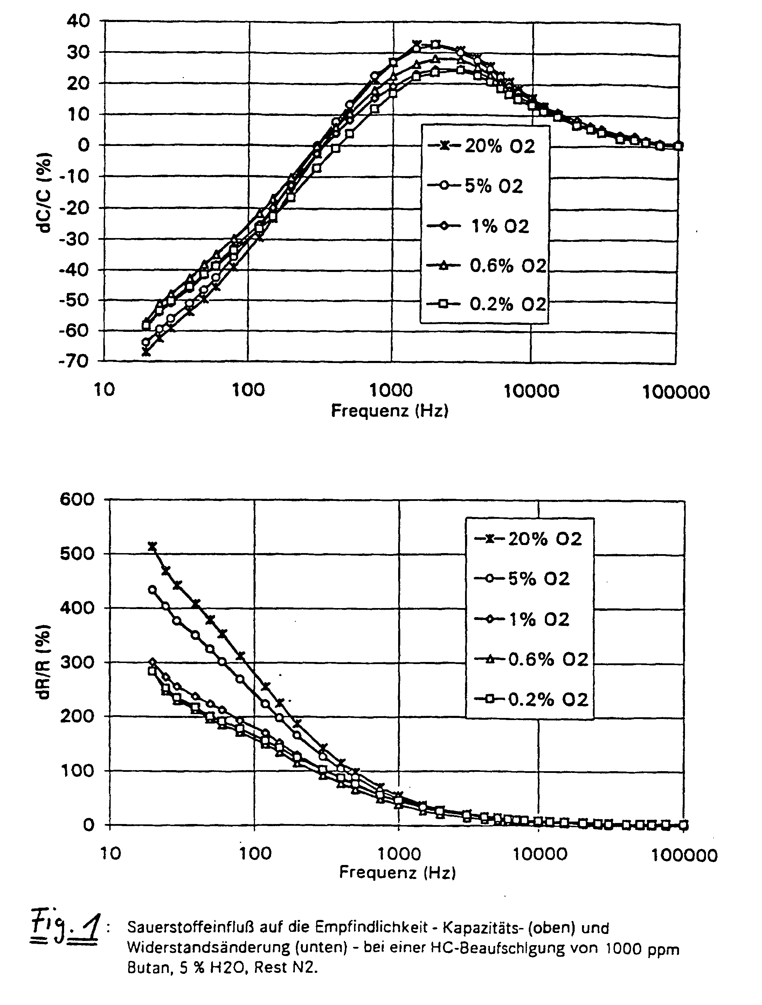

Fig. 1 zeigt die Auswirkungen der Sauerstoffkonzentrationsabnahme auf die aus der Impedanzmessung gewonnenen relativen Kapazitätsänderungen dC/C (oben) und relativen Widerstandsänderung dR/R (unten) in Abhängigkeit von der Frequenz bei einer Zugabe von 1000 ppm Butan als dem zu detektierenden Kohlenwasserstoff. An der Abszisse sind jeweils die angelegten Frequenzen aufgetragen. An der Ordinate ist der Wert dC/C bzw. dR/R in Prozent angegeben. Dabei werden C und R aus dem Imaginär- und Realteil der gemessenen komplexen Impedanz bei fester Frequenz berechnet. dC bzw. dR bezeichnet die Differenz der C-Werte bzw. R-Werte, die bei Messungen mit und ohne Butan-Beaufschlagung bei gleicher Frequenz ermittelt wurden. Dargestellt sind 5 Meßreihen, wobei der O₂-Gehalt innerhalb jeder Meßreihe konstant gehalten, jedoch von Meßreihe zu Meßreihe von 20% auf 0,2 % verringert wurde.
Wie man aus der Fig. 1, oben erkennt, macht sich im gesamten Frequenzbereich die Sauerstoffabnahme bei der Kapazitätsänderung fast nicht bemerkbar. So ist insbesondere bei niedriger Frequenz die Kapazitätsabnahme mit ca. -60% hinreichend hoch.
Die Widerstandsänderung (Fig. 1 unten) geht zwar mit Erniedrigung des Sauerstoffgehalts zurück, aber selbst bei 0,2% Sauerstoff verbleibt noch immer eine hinreichend hohe Empfindlichkeit. Der Sauerstoffeinfluß im Bereich niedriger Konzentrationen (0,2, 0,6 und 1,0%) und damit im Einsatzbereich beim Ottomotor ist sehr gering.

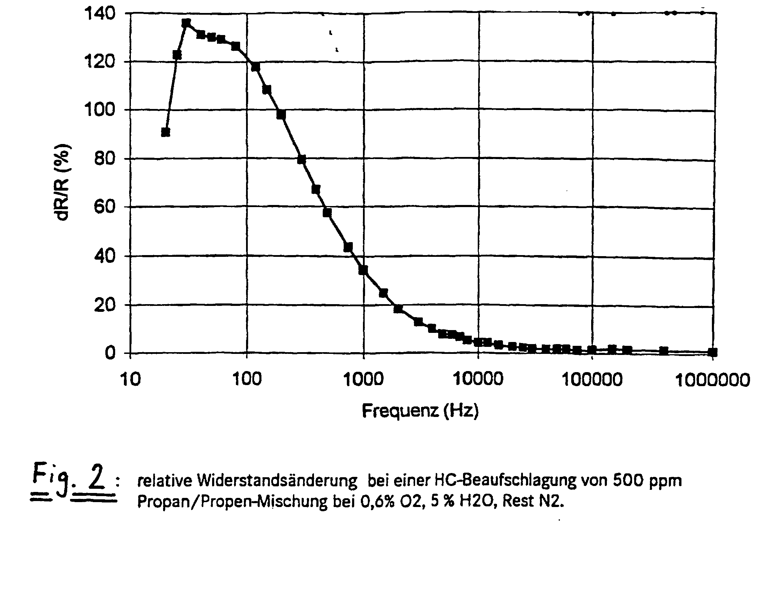

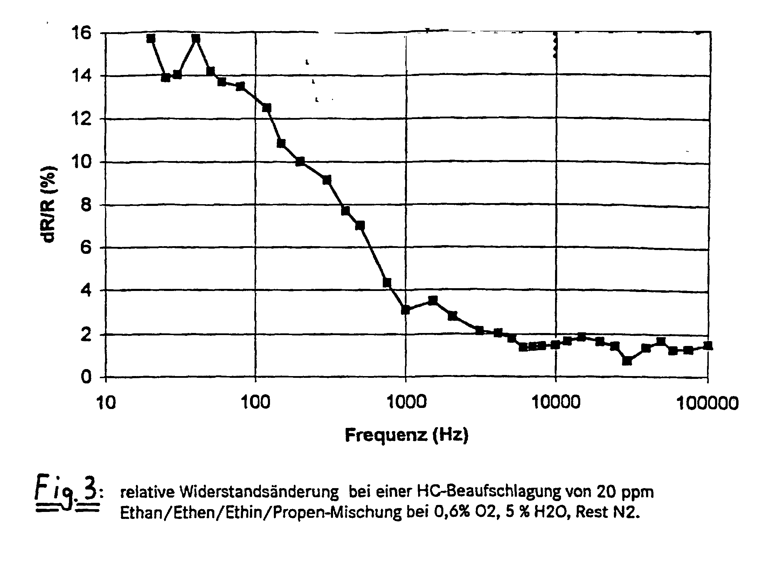

Die Fig. 2 und 3 zeigen jeweils die aus der Impedanzmessung erhaltenen Werte dR/R in Abhängigkeit von der Frequenz.
Aus Fig. 2 erkennt man, daß 500 ppm einer Propan/Propen-Mischung bei 0,6%O₂ mit hinreichender Empfindlichkeit nachgewiesen werden können. Dasselbe gilt für den Nachweis von 20 ppm einer Ethan/Ethen/Ethin/Propen-Mischung bei 0,6%O₂, wie Fig. 3 zeigt.

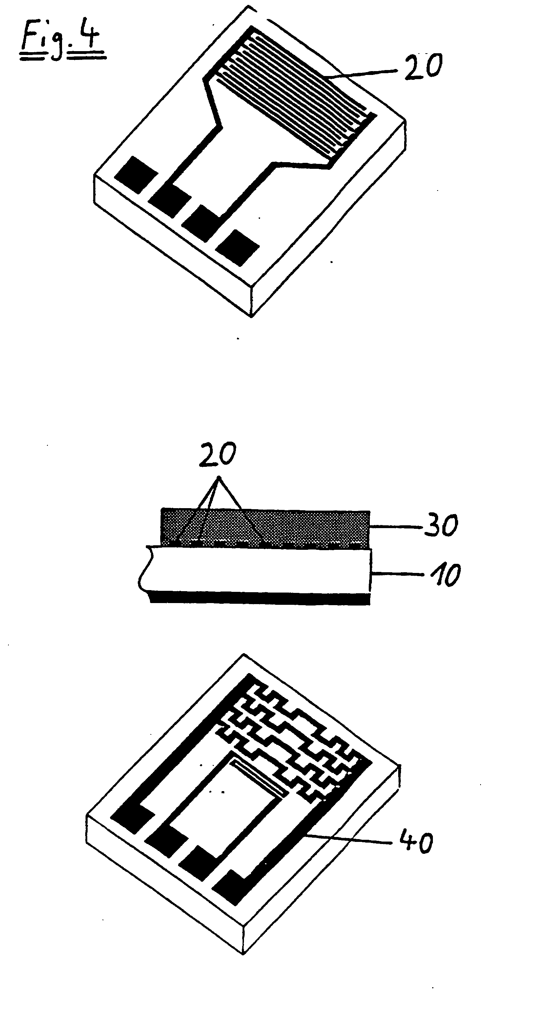

Fig. 4 zeigt einen erfindungsgemäßen Kohlenwasserstoffsensor in Form einer beschichteteten Interdigitalkondensatorstruktur. Die obere Abbildung zeigt eine Draufsicht auf die Interdigitalkondensatorstruktur. Die mittlere Abbildung zeigt einen Schnitt durch die Interdigitalkondensatorstruktur mit darauf angeordneter Zeolithschicht. Die untere Abbildung zeigt eine Draufsicht auf die zugehörige Heizerstruktur.
Auf einem Substrat **1 0**, z.B. aus Quarzglas, Si oder Al₂O₃ sind parallele Leiterbahnen **2 0** (z.B aus Au) aufgebracht, die derart verschaltet sind, daß sich eine Anordnung elektrisch parallel geschalteter Kondensatoren ergibt, wobei ein einzelner Kondensator jeweils zwei benachbarte Leiterbahnen umfaßt. Über und zwischen den Leiterbahnen **20** ist die gasdurchlässige, sensitive Zeolith-Schicht **30** angeordnet. Die Schichtdicke der Zeolithschicht **30** liegt z.B. in der Größenordnung von 40 µm. Auf der Unterseite des Substrats **10** ist eine Heizung **40** angeordnet, deren Struktur aus einzelnen Heizungsdrähten in der unteren Abbildung im Detail dargestellt ist.

### Beispiel 1

Diese Beispiel zeigt die Herstellung des Materials für die sensitive Schicht zum selektiven Nachweis von Kohlenwasserstoffen im CO und H₂-reichen und sauerstoffarmen Abgas. Im einzelnen werden folgende Verfahrensschritte durchgeführt:
a) Lösen von Pt(NH₃)4Cl₂*H₂O in destilliertem Wasser, wobei pro 1000 ml destilliertem Wasser 0,903 g Pt(NH₃)₄Cl₂*H₂O gelöst werden
b) Herstellen einer Suspension durch Zugabe eines NaZSMS-Zeolithmaterials zu der zuvor erhaltenen Lösung, wobei pro 1000 ml 50 g des Zeolithmaterials zugegeben wurde
c) Rühren der Suspension für 24 h, anschließend erfolgt ein Filtrieren, chloridfreies Waschen sowie Trocknen bei einer Trocknungstemperatur von 120 °C,
d) allmähliches Aufheizen des getrockneten, pulverförmigen Materials mit einer gleichmäßigen Rate von 4 K/min auf eine Temperatur von etwa 400 °C in H₂-haltiger Atmosphäre (z.B. 5 Vol.-% H₂ in N₂).

### Beispiel 2

Dieses Beispiel zeigt, wie das nach Beispiel 1 hergestellte Material für die sensitive Schicht auf eine Interdigitalkondensatorstruktur aufgebracht wird. Im einzelnen werden die folgenden Verfahrensschritte durchgeführt:
a) Herstellung einer siebdruckfähigen Suspension aus dem nach Beispiel 1 hergestellten Pt-Zeolithmaterial. Hierzu wird das Pt-Zeolithpulver schrittweise mit einem geeigneten Siebdruckmittel, z.B. WB41 (Fa. Zschimmer&Schwarz) im Massenverhältnis 1:1 vermischt, um eine geeignete Viskosität zu erreichen.
b) Homogenisierung der nach a) hergestellten Suspension durch zwei Läufe durch eine Walzenmühle,
c) Beschichtung der IDK-Fläche mit der nach a) und b) hergestellten Suspension mittels eines Siebdrucknetzes (z.B. Maschenzahl 100, Maschenweite 180 µm, Dicke 70 µm / 130µm). Die Aussparungsfenster des Siebdrucknetzes entsprechen den Abmessungen der IDK-Fläche.
d) Trocknung des so hergestellten Sensors für eine Stunde an Luft,
e) Ausheizen des Sensors mit eine mit einer Aufheizrate von 0,2 K/min von Raumtemperatur auf 400 °C in geeigneter Atmosphäre (z.B. Luft), um das Wasser und die organischen Bestandteile aus der Schicht herauszubringen.

## Patentansprüche

1. Gassensor zum selektiven Nachweis von Kohlenwasserstoffen im Abgasstrom von Ottomotoren, umfassend
- ein als Kondensator wirkendes Bauteil,
- eine gasdurchlässige, sensitive Schicht als Dielektrikum, wobei die sensitive Schicht ein Edelmetall-dotierter Zeolith ist, der eine geordnete, kristalline Struktur aus Primärporen aufweist, deren Durchmesser in der Größenordnung des gaskinetischen Durchmessers der zu detektierenden Gasmoleküle ist,
**dadurch gekennzeichnet,**
**dass** der Edelmetall-dotierte Zeolith ein ZSM5-Zeolith mit einem Porendurchmesser der Primärporen im Bereich von 0,53 bis 0,56 nm ist und die Edelmetalldotierung durch Pt oder Pd mit einem Anteil von 0,1 bis 5 Gew.-% am Zeolith gebildet wird.

2. Gassensor nach Anspruch 1,
**dadurch gekennzeichnet**
**dass** das als Kondensator wirkende Bauteil ein Interdigitalkondensator ist.

3. Verwendung eines Gassensors nach Anspruch 1 oder 2, in einem Abgasstrom mit einem Sauerstoffgehalt von weniger als 10000 ppm.

## Claims

1. Gas sensor for the selective detection of hydrocarbons in the exhaust-gas stream from spark ignition engines, comprising
- a component which acts as a capacitor,
- a gas-permeable, sensitive layer as dielectric, the sensitive layer being a precious metal-doped zeolite which has an ordered, crystalline structure of primary pores whose diameter is of the order of magnitude of the gas-kinetic diameter of the gas molecules to be detected, **characterized in that** the precious metal-doped zeolite is a ZSM5 zeolite with a pore diameter for the primary pores in the range from 0.53 to 0.56 nm, and the precious metal doping is formed by Pt or Pd, forming a proportion of from 0.1 to 5% by weight of the zeolite.

2. Gas sensor according to Claim 1, **characterized in that** the component acting as a capacitor is an interdigitated capacitor.

3. Use of a gas sensor according to Claim 1 or 2 in an exhaust-gas stream with an oxygen content of less than 10 000 ppm.

## Revendications

1. Capteur de gaz pour la détection sélective d'hydrocarbures dans les gaz d'échappement de moteurs Otto, comprenant:
- une composant fonctionnant comme condensateur
- une couche sensible perméable au gaz comme diélectrique, la couche sensible étant une zéolithe dopée avec un métal noble, qui présente une structure cristalline ordonnée de pores primaires dont le diamètre est de l'ordre de grandeur du diamètre cinétique de la molécule de gaz à détecter,
**caractérisé en ce que**,
la zéolithe dopée avec un métal noble est une zéolithe ZSM5 avec un diamètre de pore des pores primaires dans la plage de 0,53 à 0,56 nm et le dopage au métal noble est constitué de Pt ou de Pd avec une teneur de 0,1 à 5% en poids par rapport à la zéolithe.

2. Capteur de gaz selon la revendication 1,
**caractérisé en ce que** le composant fonctionnant comme condensateur est un condensateur interdigital.

3. Utilisation d'un capteur de gaz selon la revendication 1 ou 2 dans un flux de gaz d'échappement présentant une teneur en oxygène inférieure à 10000 ppm.
